# EUROPÄISCHE PATENTSCHRIFT

(11) **EP 0 847 384 B1**
(45) Veröffentlichungstag und Bekanntmachung des Hinweises auf die Patenterteilung: **05.04.2000**
(21) Anmeldenummer: 96929327.3
(22) Anmeldetag: 26.08.1996
(51) Int. Cl.: C07C 271/22, A01N 47/12

(54) **CARBAMOYLCARBONSÄUREAMIDE**
CARBAMOYL CARBOXYLIC ACID AMIDES
AMIDES D'ACIDE CARBAMOYL CARBOXYLIQUE

(30) Priorität: 30.08.1995 DE 19531814; 01.09.1995 DE 19532313
(43) Veröffentlichungstag der Anmeldung: 17.06.1998
(73) Patentinhaber: BASF AKTIENGESELLSCHAFT, 67056 Ludwigshafen (DE)
(72) Erfinder: WETTERICH, Frank, D-67112 Mutterstadt (DE); WAGNER, Oliver, D-66450 Bexbach (DE); EICKEN, Karl, D-67157 Wachenheim (DE); DITRICH, Klaus, D-67161 Gönnheim (DE); AMMERMANN, Eberhard, D-64646 Heppenheim (DE); LORENZ, Gisela, D-67434 Hambach (DE); STRATHMANN, Siegfried, D-67117 Limburgerhof (DE)
(86) Internationale Anmeldenummer: EP9603755
(87) Internationale Veröffentlichungsnummer: WO9708138

(56) Entgegenhaltungen:
- DE-A- 4 321 897
- DE-A- 4 431 467

## Beschreibung

Die vorliegende Erfindung betrifft Carbamoylcarbonsäureamide der allgemeinen Formel I 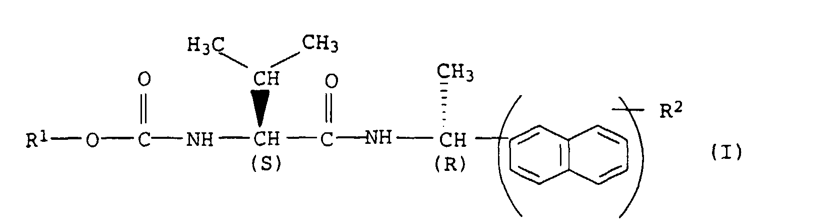 in einer Isomerenreinheit von mehr als 90 Gew.-%, in denen die Variablen die folgende Bedeutung haben:
- R¹: C₁-C₈-Alkyl, C₂-C₈-Alkenyl oder C₂-C₈-Alkinyl,
wobei diese Reste partiell oder vollständig halogeniert sein und/oder eine bis drei der folgenden Gruppen tragen können: Cyano, C₁-C₄-Alkoxy, C₁-C₄-Halogenalkoxy, C₁-C₄-Alkylthio, C₁-C₄-Alkoxycarbonyl, C₃-C₇-Cycloalkyl, C₃-C₇-Cycloalkenyl, Aryl, Aryloxy und Heteroaryl,
wobei die cyclischen und aromatischen Ringe dieser Gruppen ihrerseits einen bis drei der folgenden Substituenten tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl, Aryloxy und Heteroaryl;
- R²: Wasserstoff, Halogen, Cyano, Nitro, C₁-C₈-Alkyl, C₁-C₄-Alkoxyalkyl, C₁-C₄-Halogenalkyl, C₁-C₄-Alkoxy, C₁-C₄-Haloqenalkoxy, C₁-C₄-Alkylthio, C₁-C₄-Halogenalkylthio oder eine über Sauerstoff oder Schwefel gebundene Phenylgruppe, welche unsubstituiert ist oder einen bis drei der folgenden Substituenten tragen kann: Halogen, C₁-C₄-Alkyl und C₁-C₄-Alkoxy.

(S) steht für die S-Konfiguration, (R) für die R-Konfiguration des so markierten asymmetrischen Kohlenstoffatoms gemäß der IUPAC-Nomenklatur. Insbesondere entspricht die Konfiguration des S-Zentrums in den Verbindungen der allgemeinen Formel I jener des i L-Valins. Die Konfiguration der Verbindungen I wird der Einfachheit halber im folgenden als (SR)-Konfiguration bezeichnet.

Die "Isomerenreinheit" bezeichnet den Prozentanteil einer Verbindung I (Konfiguration (SR)) an der Gesamtheit der möglichen vier Diastereomeren dieser Verbindungen I ((SR), (RS), (RR), (SS)).

Außerdem betrifft die Erfindung Verfahren zur Herstellung der Verbindungen I. Die Erfindung betrifft weiterhin Mittel, welche die Verbindungen I enthalten, ein Verfahren zur Herstellung derartiger Mittel sowie ein Verfahren zur Bekämpfung von Schadpilzen und die Verwendung der Verbindungen I oder der Mittel hierzu.

Racemische Gemische von fungiziden Verbindungen vom Typ I sind vor allem aus der DE-A 43 21 897 und der älteren deutschen Anmeldung P 44 31 467.1 bekannt.

Diese Gemische befriedigen jedoch hinsichtlich ihrer fungiziden Wirkung noch nicht.

Der vorliegenden Erfindung lagen daher neue Carbamoylcarbonsäureamide hoher Isomerenreinheit mit verbesserter Wirkung gegen Schadpilze als Aufgabe zugrunde.

Demgemäß wurden die eingangs definierten Verbindungen I gefunden sowie sie enthaltende Mittel gefunden.

Ferner wurden Verfahren zur Herstellung der Verbindungen I und der sie enthaltenden Mittel gefunden und des weiteren ein Verfahren zur Bekämpfung von Schadpilzen und die Verwendung der Verbindungen I oder der Mittel hierzu.

Die Verbindungen I können in an sich bekannter Weise ausgehend von den entsprechenden, auf L-Valin basierenden, Carbamoylcarbonsäuren II hergestellt werden. Bevorzugt erhält man die Verbindungen I nach den im folgenden beschriebenen Verfahren A bzw. B (die Literaturzitate "Houben-Weyl" beziehen sich auf: Houben-Weyl, Methoden der Organischen Chemie, 4. Auflage, Thieme Verlag, Stuttgart).

### Verfahren A

Die Carbamoylcarbonsäureamide I, erhält man, indem man die Carbamoylcarbonsäuren II mit den Aminen III umsetzt.

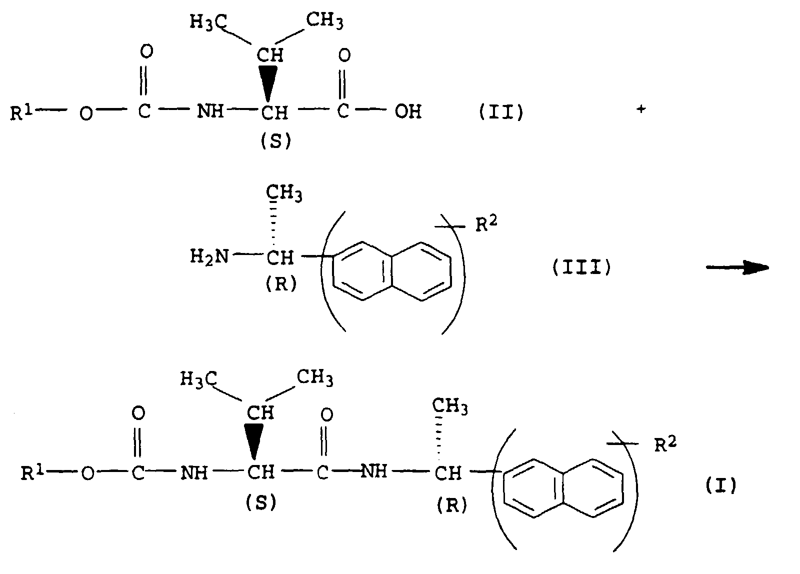

Die Carbamoylcarbonsäuren II sind bekannt oder können nach bekannten Methoden, vor allem ausgehend von der Aminosäure L-Valin hergestellt werden (vgl. "Houben-Weyl", Band 15/1, Seite 46 bis Seite 305, vor allem Seite 117 bis Seite 125).

Die Amine III sind ebenfalls bekannt oder können leicht erhalten werden (vgl. Organikum, VEB Deutscher Verlag der Wissenschaften, 15. Auflage, Berlin, 1977, Seite 610 ff.; "Houben-Weyl", Band 15/1, Seiten 648-665; Indian J. Chem. 10, Seite 366 (1972); J. Am. Chem. Soc. 58, Seiten 1808-1811 (1936)).

Aus Racematen der Amine III kann das R-Isomere in an sich bekannter Weise, etwa durch fraktionierte Kristallisation mit optisch aktiver Weinsäure oder vorzugsweise mittels enzymkatalysierter Veresterung und anschließender Verseifung separiert werden (vgl. z.B. die WO-A 95/08636).

Vorzugsweise arbeitet man in diesem Verfahren A so, daß man zunächst die Carbamoylcarbonsäuren II in carboxyaktivierte Derivate, vor allem in Acylcyanide oder Anhydride, überführt (vgl. Tetrahedron Letters, Band 18, Seite 1595 bis Seite 1598 (1973), bzw. "Houben-Weyl", Band 15/1, Seite 28 bis Seite 32). Diese Derivate werden dann mit den Aminen III in Gegenwart von Basen zur Reaktion gebracht.

Zur Herstellung der carboxyaktivierten Acylcyanide eignet sich z.B. die Reaktion der Carbamoylcarbonsäuren II mit Cyanphosphonsäurediethylester, vor allem in einem inerten Lösungsmittel wie Tetrahydrofuran oder Toluol.

Zur Herstellung der carboxyaktivierten Anhydride ist die Umsetzung der Carbamoylcarbonsäure II mit Kohlensäurechloriden wie Chlorameisensäure-iso-butylester in Gegenwart von Basen und gegebenenfalls in einem inerten Lösungsmittel wie Toluol oder Tetrahydrofuran bevorzugt.

Die Umsetzung der Amine III mit den carboxyaktivierten Carbamoylcarbonsäuren II erfolgt vorzugsweise in einem Lösungsmittel wie Dichlormethan, Tetrahydrofuran oder Toluol.

Als Basen können auch die Amine III dienen, wobei man sie üblicherweise aus dem Rohprodukt üblicherweise zurückgewinnt.

In einer bevorzugten Ausführungsform dieser Verfahrensstufe werden die Carbamoylcarbonsäure II, das Amin III, das zur Erzeugung des carboxyaktivierten Derivates der Carbamoylcarbonsäure II geeignete Reagens und die Base im Eintopfverfahren, gegebenenfalls in einem inerten Lösungsmittel, zur Reaktion gebracht und das Rohprodukt anschließend in an sich bekannter Weise auf das Carbamoylcarbonsäureamid I aufgearbeitet.

### Verfahren B

Die Carbamoylcarbonsäureamide I, erhält man, indem man die Carbamoylcarbonsäureamide I, in denen die Gruppe R¹-O-(CO) für eine Schutzgruppe steht, die in an sich bekannter Weise abgespalten werden kann, in Aminosäureamide IV überführt und diese mit Chlorameisensäureestern V in Gegenwart einervon Basen umsetzt.

### Stufe Ba: Herstellung der Aminosäureamide IV

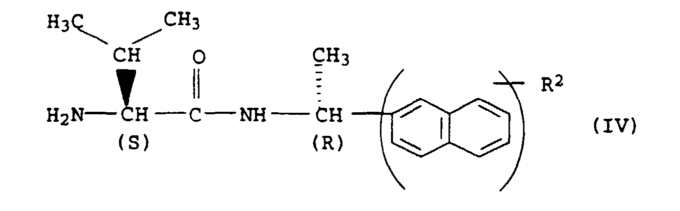

Die Abspaltung der Gruppe R¹-O-(CO) aus den Carbamoylcarbonsäureamiden I kann in an sich bekannter Weise durchgeführt werden (vgl. "Houben-Weyl", Band 15/1, Seite 46 bis Seite 305, vor allem Seite 126 bis Seite 129).

Geeignete abspaltbare Gruppen enthalten als Rest R¹ die tert.-Butyl- oder und daneben die Benzylgruppe.

Im Falle von R¹ = tert.-Butyl beispielsweise erfolgt die Abspaltung üblicherweise durch Umsetzung mit einer Säure, insbesondere einer Protonensäure wie Salzsäure oder Trifluoressigsäure (ibid., Seite 126 bis Seite 129).

Die als Ausgangsstoffe geeigneten Carbamoylcarbonsäureamide I können nach bekannten Verfahren (vgl. "Houben-Weyl", Band 15/1, Seite 28 bis Seite 32) oder insbesondere nach dem erfindungsgemäßen Verfahren A gewonnen werden.

### Stufe Bb: Herstellung der Carbamoylcarbonsäureamide I

Die aus der Synthesestufe (Ba) resultierenden Aminosäureamide IV werden mit den Chlorameisensäureestern V in Gegenwart von Basen umgesetzt.

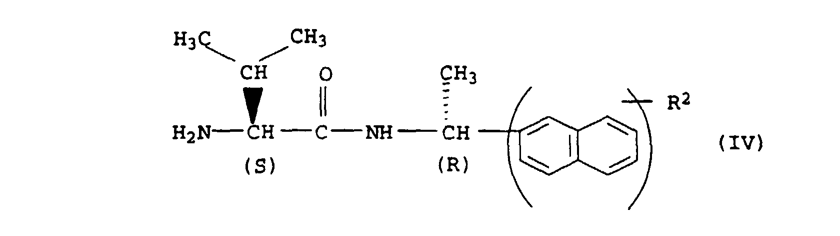

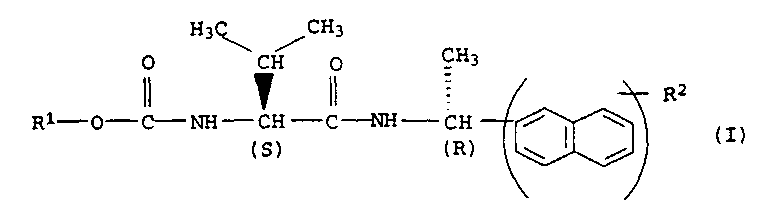

Die Chlorameisensäureester V sind allgemein bekannt oder können nach bekannten Verfahren hergestellt werden.

Die Umsetzung wird vorzugsweise in einem organischen Lösungsmittel, vor allem Toluol, Methylenchlorid oder Tetrahydrofuran, oder Gemischen dieser Lösungsmittel durchgeführt.

Als Basen kommen anorganische und organische Basen gleichermaßen in Betracht, wobei organische Basen und hierunter wiederum tertiäre Amine wie Triethylamin, Pyridin und N-Methylpiperidin bevorzugt sind.

Die Umsetzung wird in der Regel bei Temperaturen von (-40) bis 50, vorzugsweise (-10) bis 20°C durchgeführt.

Im übrigen ist die Durchführung dieser Reaktion dem Fachmann geläufig, so daß es keiner weiteren Ausführungen hierzu bedarf (vgl. "Houben-Weyl", Band 15/1, Seite 117 bis Seite 139).

Die nach den Verfahren A bzw. B erhaltenen Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit werden können. Sofern die Zwischen- und Endprodukte als Feststoffe erhalten werden, kann die Reinigung auch beispielsweise durch Umkristallisieren oder Digerieren erfolgen.

Bei der eingangs angegebenen Definition der Verbindungen I wurden Sammelbegriffe verwendet, die repräsentativ für die folgenden Substituenten stehen:
Halogen: Fluor, Chlor, Brom und Jod;
   Alkyl: geradkettige oder verzweigte Alkylgruppen mit 1 bis 8 Kohlenstoffatomen, z.B. C₁-C₆-Alkyl wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 2,2-Dimethylpropyl, 1,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl bzw. partiell oder vollständig halogeniertes Alkyl: geradkettige oder verzweigte Alkylgruppen mit 1 bis 4 bzw. 8 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkoxygruppen mit 1 bis 4 Kohlenstoffatomen, z.B. C₁-C₃-Alkoxy wie Methyloxy, Ethyloxy, Propyloxy und 1-Methylethyloxy;
Alkoxyalkyl: geradkettige oder verzweigte Alkylgruppen mit 1 bis 8 Kohlenstoffatomen (wie vorstehend genannt), welche in einer beliebigen Position eine geradkettige oder verzweigte Alkoxygruppe (wie vorstehend genannt) mit im Falle von C₁-C₄-Alkoxyalkyl 1 bis 4 Kohlenstoffatomen tragen, wie Methoxymethyl, Ethoxymethyl, n-Propoxymethyl, n-Butoxymethyl, 1-Methoxyethyl, 2-Methoxyethyl, 1-Ethoxyethyl, 2-Ethoxyethyl, 2-n-Propoxyethyl und 2-Butoxyethyl;
Halogenalkoxy: geradkettige oder verzweigte Alkoxygruppen mit 1 bis 4 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkoxy wie Chlormethyloxy, Dichlormethyloxy, Tri-chlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-di-fluorethyloxy, 2,2-Dichlor-2-fluorethyloxy, 2,2,2-Trichlorethyloxy und Pentafluorethyloxy;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind, z.B. C₁-C₄-Alkylthio wie Methylthio, Ethylthio, Propylthio, 1-Methylethylthio, n-Butylthio und tert.-Butylthio;
Alkoxycarbonyl: geradkettige oder verzweigte Alkoxygruppen mit 1 bis 4 C-Atomen (wie vorstehend genannt), welche über eine Carbonylgruppe (-CO-) an das Gerüst gebunden sind;
Alkenyl: geradkettige oder verzweigte Alkenylgruppen mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 2-Methyl-1-propenyl, 1-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, l,2-Dimethyl-3-butenyl, 1,3-Dimethyl-1-butenyl, 1,3-Dimethyl-2-butenyl, 1,3-Dimethyl-3-butenyl, 2,2-Dimethyl-3butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, l-Ethyl-2-methyl-1-propenyl und 1-Ethyl-2-methyl-2-propenyl;
Alkinyl: geradkettige oder verzweigte Alkinylgruppen mit 2 bis 8 Kohlenstoffatomen und einer Dreifachbindung in einer beliebigen Position, z.B. C2-C6-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und l-Ethyl-1-methyl-2-propinyl;
Cycloalkyl: monocyclische Alkylgruppen mit 3 bis 7 Kohlenstoffringgliedern, z.B. C₃-C₇-Cycloalkyl wie Cyclopropyl, Cyclobutyl, Cyclopentyl, Cyclohexyl und Cycloheptyl;
Cycloalkenyl: monocyclische Alkylgruppen mit 5 bis 7 Kohlenstoffringgliedern die eine oder mehrere Doppelbindungen enthalten z.B. C₅-C₇-Cycloalkenyl wie Cyclopentenyl, Cyclohexenyl und Cycloheptenyl;
Aryloxy: Arylgruppen (wie vorstehend genannt), welche über ein Sauerstoffatom (-O-) an das Gerüst gebunden sind wie Phenoxy, 1-Naphthoxy und 2-Naphthoxy;
Heteroaryl: aromatische mono- oder polycyclische Reste, welche neben Kohlenstoffringgliedern zusätzlich 1 bis 4 Stickstoffatome oder 1 bis 3 Stickstoffatome und ein Sauerstoff- oder ein Schwefelatom oder ein Sauerstoff- oder ein Schwefelatom enthalten können, z.B.:
   - 5-gliedriges Heteroaryl, enthaltend 1 bis 3 Stickstoffatome: 5-Ring-Heteroarylgruppen, welche neben Kohlenstoffatomen 1 bis 3 Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend 1 bis 4 Stickstoffatome oder 1 bis 3 Stickstoffatome und 1 Schwefelatom oder Sauerstoffatom oder 1 Sauerstoff- oder 1 Schwefelatom: 5-Ring-Heteroarylgruppen, welche neben Kohlenstoffatomen 1 bis 4 Stickstoffatome oder 1 bis 3 Stickstoffatome und 1 Schwefel-oder Sauerstoffatom oder 1 Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, l,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
   - benzokondensiertes 5-gliedriges Heteroaryl, enthaltend 1 bis 3 Stickstoffatome oder 1 Stickstoffatom und/oder ein Sauerstoff- oder Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder ein Schwefelatom als Ringglieder enthalten i können, und in welchen 2 benachbarte Kohlenstoffringglieder oder 1 Stickstoff- und 1 benachbartes Kohlenstoffringglied durch eine Buta-1,3-dien-1,4-diylgruppe verbrückt sein können;
   - über Stickstoff gebundenes 5-gliedriges Heteroaryl, enthaltend 1 bis 4 Stickstoffatome, oder über Stickstoff gebundenes benzokondensiertes 5-gliedriges Heteroaryl, enthaltend 1 bis 3 Stickstoffatome: 5-Ring-Heteroarylgruppen, welche neben Kohlenstoffatomen 1 bis 4 Stickstoffatome bzw. 1 bis 3 Stickstoffatome als Ringglieder enthalten können, und in welchen 2 benachbarte Kohlenstoffringglieder oder ein Stickstoff- und ein benachbartes Kohlenstoffringglied durch eine Buta-1,3-dien-l,4-diylgruppe verbrückt sein können, wobei diese Ringe über eines der Stickstoffringglieder an das Gerüst gebunden sind;
   - 6-gliedriges Heteroaryl, enthaltend 1 bis 3 bzw. 1 bis 4 Stickstoffatome: 6-Ring-Heteroarylgruppen, welche neben Kohlenstoffatomen 1 bis 3 bzw. 1 bis 4 Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedriges Heteroaryl, enthaltend 1 bis 4 Stickstoffatome: 6-Ring-Heteroarylgruppen, in welchen 2 benachbarte Kohlenstoffringglieder durch eine Buta-1,3-dien-1,4-diylgruppe verbrückt sein können, z.B. Chinolin, Isochinolin, Chinazolin und Chinoxalin.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

Im Hinblick auf ihre Wirkung gegen Schadpilze sind Verbindungen I bevorzugt, welche eine Isomereneinheit von mindestens 93, und insbesondere von mindestens 95 % aufweisen.

Im Hinblick auf ihre Wirkung gegen Schadpilze sind ferner Verbindungen I bevorzugt, in denen die Reste R¹ und R² die folgenden Bedeutungen haben, und zwar für sich allein oder in Kombination. Die in den Restebedeutungen im folgenden genannten Gruppen können auch anspruchsgemäß substituiert sein.
- R¹: C₁-C₈-Alkyl, vorzugsweise C₁-C₄-Alkyl und insbesondere iso-Propyl, tert.-Butyl oder sec.-Butyl;
- R²: Wasserstoff, Halogen, Cyano, C₁-C₄-Alkyl oder C₁-C₄-Alkoxy, vorzugsweise Wasserstoff, Chlor, Cyano, Methyl oder Methoxy und insbesondere Wasserstoff.

Ganz besonders bevorzugt sind im Hinblick auf ihre Verwendung die in den anschließenden Tabellen 1 und 2 zusammengestellten Verbindungen I.

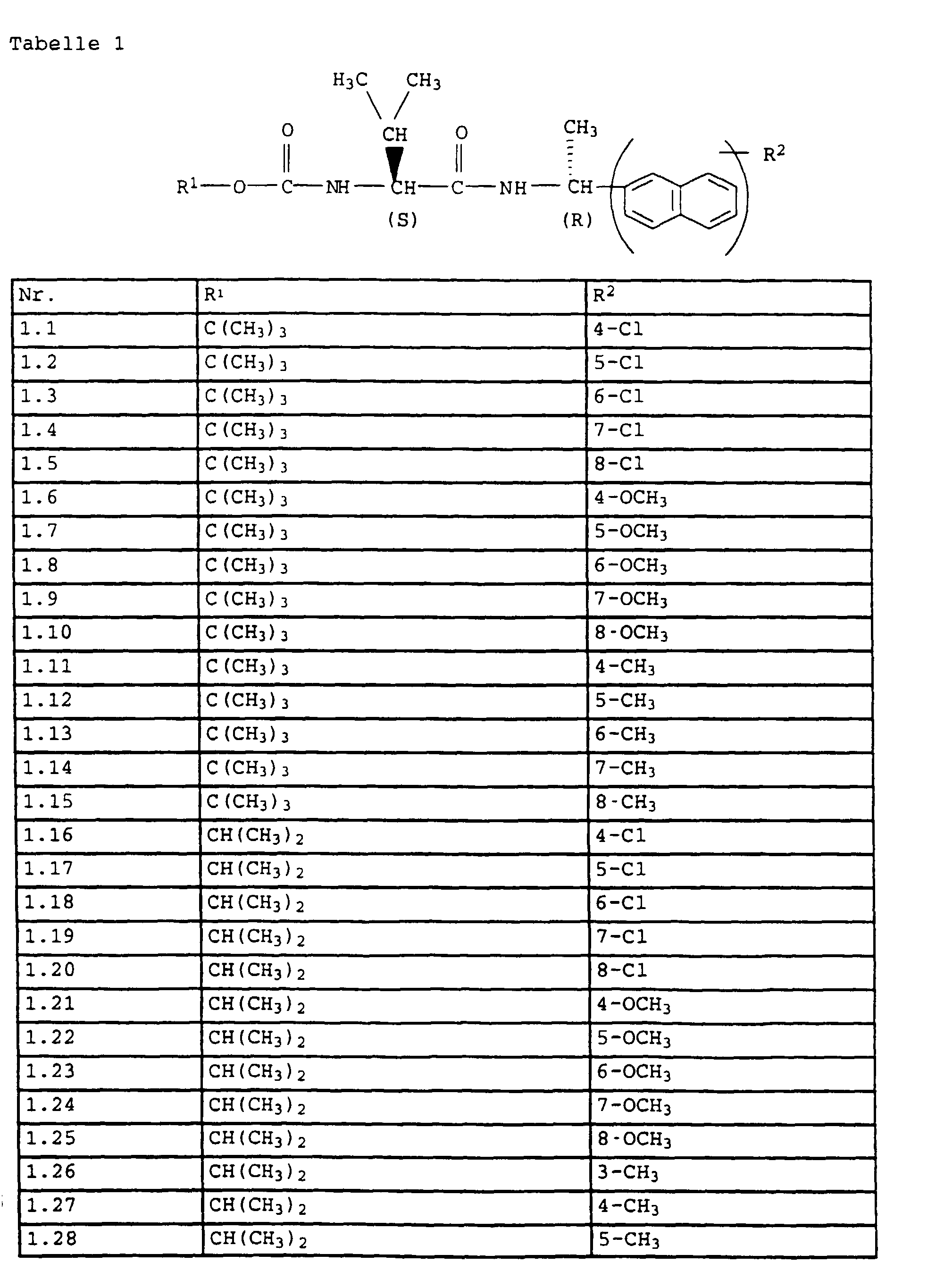

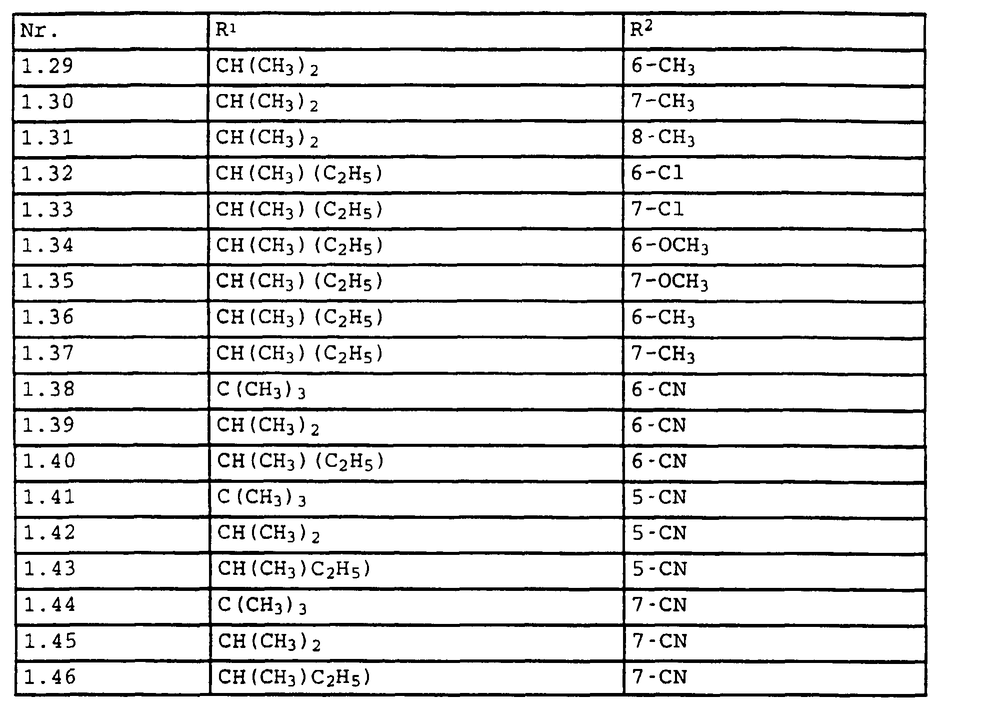

Aus den in Tabelle 1 genannten Verbindungen sind wiederum jene bevorzugt, in welchen der Substituent R² in der 5- oder 6-Position des Naphthalin-Ringsystems steht.

Die neuen Verbindungen der Formel I eignen sich zur Bekämpfung von Schadpilzen.

Die neuen Verbindungen I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden bei der Pflanzenbehandlung die Pflanzen mit den Wirkstoffen besprüht oder bestäubt oder die Samen der Pflanzen mit den Wirkstoffen behandelt.

Die Formulierungen werden unter Verwendung üblicher Formulierungshilfsmittel - wie im folgenden ausgeführt wird - und in an sich bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta-und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylen-alkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalk; stein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-isobutylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen aus, und zwar insbesondere aus der Klasse der Phycomyceten und daneben der Deuteromyceten, Ascomyceten und Basidiomyceten. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Schadpilze, deren Lebensraum oder die von ihnen freizuhaltenden Pflanzen, Räume, Flächen oder Materialien mit einer wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:
Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Pseudoperenospora cubensis an Gurken, Fusarium- und Verticillium-Arten an verschiedenen Pflanzen, Plasmopara viticola an Reben, Pseudoperenospora humuli an Hopfen und Alternaria-Arten an Gemüse und Obst.
   Die neuen Verbindungen können auch im Materialschutz (Holzschutz) eingesetzt werden, z.B. gegen Paecilomyces variotii.
   Die fungiziden Mittel enthalten im allgemeinen zwischen 0,1 und 95, vorzugsweise zwischen 0,5 und 90 Gew.-% Wirkstoff.
   Die Aufwandmengen liegen je nach Art des gewünschten Effektes zwischen 0,025 und 2, vorzugsweise 0,1 bis 1 kg Wirkstoff pro ha.
   Bei der Saatgutbehandlung werden im allgemeinen Wirkstoffmengen von 0,001 bis 50, vorzugsweise 0,01 bis 10 g je Kilogramm Saatgut benötigt.
   Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.
   Beim Vermischen mit Fungiziden erhält man dabei in vielen Fällen eine Vergrößerung des fungiziden Wirkungsspektrums.
   Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, AmmoniakKomplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-l,2,4-triazol, 2,3-Dicyano-1,4-dithioanthrachinon, 2-Thio-1,3-dithiolo [4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-l,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethylfuran-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan,
2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]1H-1,2,4-triazol 1-[2-(2,4-Dichlorphenyl)-4-n-propyl-1,3-di-oxolan-2-yl-ethyl]-1H-l,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl]-1,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[α-(o-tolyloxy)-o-tolyl]acetat, Methyl-E-2-{2-[6-{2-cyanophenoxy)pyridimin-4-yloxy]phenyl}-3-methoxyacrylat, Methyl-E-methoximino-[α-(2,5-di-methyloxy)-o-tolyl]acetamid.

Anilino-Pyrimidine wie N-(4,6-dimethylpyrimidin-2-yl)anilin, N- [4-methyl-6-(1-propinyl)pyrimidin-2-yl]anilin, N-(4-methyl-6-cyclopropyl-pyrimidin-2-yl)anilin.

Phenylpyrrole wie 4-(2,2-difluor-1,3-benzodioxol-4-yl)pyrrol-3-carbonitril.

Zimtsäureamide wie 3-(4-chlorphenyl)-3-(3,4-dimethoxyphenyl)acrylsäuremorpholid.

### Synthesebeispiel

Die im nachstehenden Synthesebeispiel wiedergegebene Vorschrift kann unter Abwandlung der Ausgangsverbindung zur Gewinnung weiterer Vertreter der Verbindungen I benutzt werden. Die physikalischen Daten der demgemäß hergestellten Produkte sind in der anschließenden Tabelle 2 wiedergegeben.
1. (R)-1-Amino-1-(β-naphthyl)ethan
   1.1 Herstellung von (R)-Methoxyessigsäure-1-(β-naphthyl)ethylamid 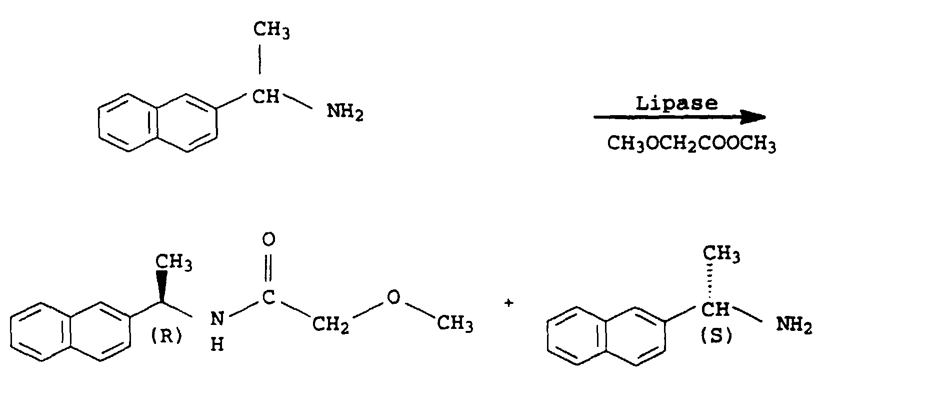 39 g (0,23 mol) racemisches 1-Amino-1-(β-naphthyl)ethan wurden in 200 ml Methyl-tert.-butylether gelöst. Die Lösung wurde mit 29,5 g (0,25 mol) Methoxyessigsäuremethylester versetzt, die Reaktion durch Zusatz von 0,5 g Lipase (ca. 1000 U/mg, Pseudomonas spec. DSM 8246) gestartet und der Reaktionsansatz während der Umsetzung auf einem Rütteltisch durchmischt. Nach Erreichen eines Umsatzes von 50% (Überprüfung mittels Gaschromatographie), was nach ca. 48 h erreicht war, wurde das Enzym abfiltriert. Das Filtrat wurde eingeengt und mit verdünnter Salzsäure (300 ml) und Diethylether (300 ml) aufgenommen. Nach Abtrennen der etherischen Phase wurde die saure Phase nochmals mit Diethylether extrahiert. Nach Vereinigen, Trocknen und Einengen der Etherphasen erhielt man 18,7 g (0,08 mol) (R)-Methoxyessigsäure-1-(β-naphthyl) ethylamid. Aus der wäßrigen Phase ließ sich nach Zugabe von Natronlauge bis zum alkalischen pH-Wert das (S)-1-Amino-1-(β-naphthyl)ethan mit Diethylether extrahieren. Trocknen und Einengen der organischen Phase ergaben 15 g (0,09 mol) (S)-1-Amino-1-(β-naphthyl)ethan. Der Enantiomerenüberschuß (=ee) wurde nach Umsetzung zum Trifluoracetamid auf einer chiralen GC-Säule (20 m Chiralolex B-Ph) zu 89,5 % bestimmt.
   1.2 Hydrolyse von (R)-Methoxyessigsäure-1-(β-naphthyl)ethylamid 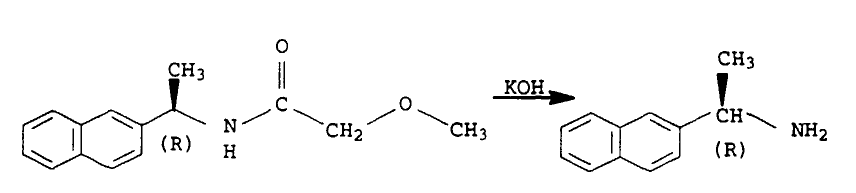 14,7 g (60,4 mmol) (R)-Methoxyessigsäure-1-(β-naphthyl)ethylamid wurden in 75 ml Ethylenglykol gelöst und hierzu 15 g 50%-ige Kaliumhydroxidlösung gegeben. Nach 3-stündigem Erhitzen auf 150°C wurde abgekühlt, mit 300 ml Wasser verdünnt und viermal mit je 500 ml Diethylether extrahiert. Die vereinigten Etherphasen wurden getrocknet und eingeengt. Man erhielt 8,1 g (47 mmol) (R)-1-Amino-1-(β-naphthyl)ethan mit einem ee-Wert von 94,8 %.
2. N-(tert.-Butyloxycarbonyl)-L-valin-(R)-1-(-naphthyl)ethylamid
   Zu der Lösung von 1,2 g (5,8 mmol) tert.-Butoxycarbonyl-L-valin und 1,0 g (5,8 mmol) (R)-1-Amino-1-(β-naphthyl)ethan in 50 ml Tetrahydrofuran wurden 1,0 g (5,9 mmol) Cyanphosphorsäurediethylester und 1,3 g (12 mmol) Triethylamin zugegeben. Es wurde eine Stunde bei 0°C und 15 Stunden bei 20°C nachgerührt. Anschließend wurde das Lösungsmittel entfernt und der Rückstand mit 300 ml Essigsäureethylester aufgenommen. Die organische Phase wurde nacheinander mit jeweils 200 ml 5%-iger Natronlauge, 10%-iger Salzsäure, 10%-iger Natriumhydrogencarbonatlösung und Wasser gewaschen, getrocknet und eingeengt. Es verblieben 2,0 g (5,4 mmol) N-(tert.-Butyloxycarbonyl)-L-valin-(R)-1-(β-naphthyl)ethylamid (Fp. 93°C, Verbindung 2.1 in Tabelle 2)
3. N-(iso-Propyloxycarbonyl)-L-valin-(R)-1-(β-naphthyl)ethylamid
   Zu 1,70 g (4,6 mmol) N-(tert.-Butyloxycarbonyl)-L-valin-(R)-1-(β-naphthyl)ethylamid wurden unter Kühlung 5 ml Trifluoressigsäure gegeben und die Mischung 1 Stunde bei 0°C gerührt. Anschließend wurde auf 20°C erwärmt, die Trifluoressigsäure weitgehend abdestilliert, der Rückstand in 100 ml Dichlormethan aufgenommen und nacheinander mit je 50 ml 2n Natronlauge, 5%-iger Natriumhydrogencarbonatlösung sowie Wasser gewaschen. Nach Trocknen und Einengen der organischen Phase verblieben 1,07 g (4,0 mmol) L-Valin-(R)-1-(β-naphthyl)ethylamid als gelbes zähflüssiges Öl.

0,54 g (2,0 mmol) dieser Verbindung und 0,22 g (2,2 mmol) Triethylamin in 40 ml Toluol wurden bei 0°C mit 0,24 g (2,1 mmol) Chlorameisensäure-iso-propylester versetzt und 15 Stunden bei 20°C gerührt. Nach Entfernen des Lösungsmittels wurde der Rückstand mit 50 ml Essigsäureethylester aufgenommen und nacheinander mit jeweils 40 ml 5%-iger Natronlauge, 10%-iger Salzsäure, 10%-iger Natriumhydrogencarbonatlösung und Wasser gewaschen. Nach Trocknen der organischen Phase wurde das Lösungsmittel entfernt. Es verblieben 0,6 g (1,7 mmol) der Titelverbindung als farbloser kristalliner Rückstand (Fp. 145-7°C, Verbindung 2.2 in Tabelle 2).

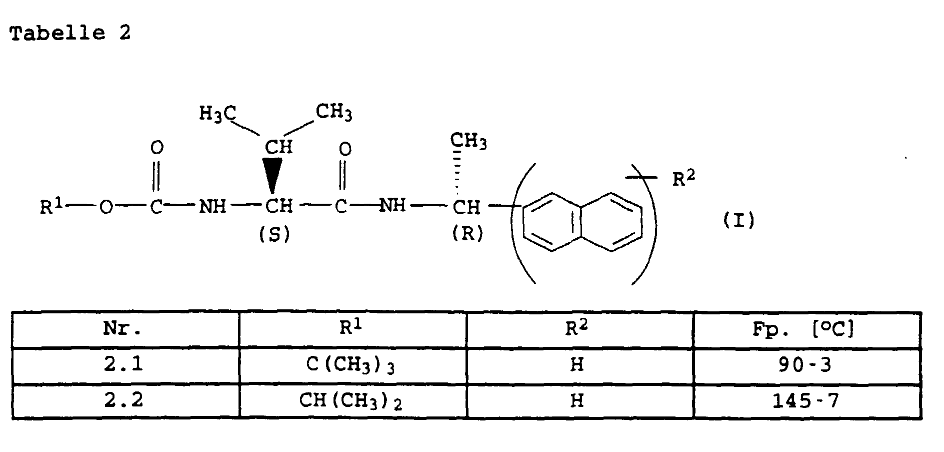

### Anwendungsbeispiele

Für die folgenden Versuche, welche die fungizide Wirkung der Verbindungen I zeigen sollen, wurde eine Emulsion verwendet, welche zu 10 Gew.-% aus dem Wirkstoff der allgemeinen Formel I, und zu 90 Gew.-% aus einem Gemisch aus
- 70 Gew.-% Cyclohexanol,
- 20 Gew.-% Nekanil® LN (Lutensol® AP6, Netzmittel mit Emulgier- und Dispergierwirkung auf der Basis ethoxylierter Alkylphenole) und
- 10 Gew.-% Emulphor® EL (Emulan® EL, Emulgator auf der Basis ethoxylierter Fettalkohole)
bestand. Die gewünschte Wirkstoff-Konzentration wurde durch Verdünnen dieser Emulsion mit Wasser eingestellt.

### Plasmopara viticola

Blätter von Topfreben der Sorte "Müller-Thurgau" wurden mit wäßriger Spritzbrühe, welche wie oben beschrieben hergestellt worden war, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Die Reben wurden zunächst für 48 Stunden in einer Kammer mit wasserdampfgesättigter Luft bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die visuelle Beurteilung des Ausmaßes des Pilzausbruchs auf den Blattunterseiten. Die Ergebnisse dieser Versuche sind der folgenden Tabelle 3 zu entnehmen

**Tabelle 3**

| Ergebnisse der Versuche mit erfindungsgemäßen Verbindungen im Vergleich zu den sie enthaltenden Racematen (bekannt aus DE-A 43 21 897) bei Plasmopara viticola | | | | |
|---|---|---|---|---|
| Wirkstoff | Befallene Blattunterseitenfläche (%) bei einer angewandten Wirkstoffkonzentration von: | | | |
| | 63 ppm | 16 ppm | 4 ppm | 1 ppm |
| 2.1 | 0 | 3 | 5 | 15 |
| Racemat zu 2.1 | 0 | 15 | 40 | - |
| 2.2 | 0 | 0 | 0 | 0 |
| Racemat zu 2.2 | 0 | 3 | 25 | 40 |

Blätter von Pflanzen, welche nicht mit einer der genannten Verbindungen behandelt worden waren, wiesen auf 75 % der Blattunterseiten-Fläche Pilzbefall auf.

## Patentansprüche

1. Carbamoylcarbonsäureamide der allgemeinen Formel I 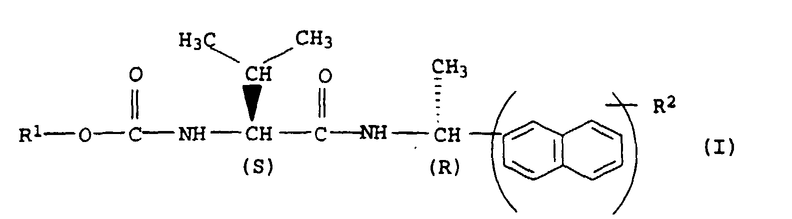 in einer Isomerenreinheit von mehr als 90 Gew.-%, in denen die Variablen die folgende Bedeutung haben:
R¹ C₁-C₈-Alkyl, C₂-C₈-Alkenyl oder C₂-C₈-Alkinyl,
wobei diese Reste partiell oder vollständig halogeniert sein und/oder eine bis drei der folgenden Gruppen tragen können: Cyano, C₁-C₄-Alkoxy, C₁-C₄-Halogenalkoxy, C₁-C₄-Alkylthio, C₁-C₄-Alkoxycarbonyl, C₃-C₇-Cycloalkyl, C₃-C₇-Cycloalkenyl, Aryl, Aryloxy und Heteroaryl,
wobei die cyclischen und aromatischen Ringe dieser Gruppen ihrerseits einen bis drei der folgenden Substituenten tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl, Aryloxy und Heteroaryl;
R² Wasserstoff, Halogen, Cyano, Nitro, C₁-C₈-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Halogenalkylthio oder eine über Sauerstoff oder Schwefel gebundene Phenylgruppe, welche unsubstituiert ist oder einen bis drei der folgenden Substituenten tragen kann: Halogen, C₁-C₄-Alkyl und C₁-C₄-Alkoxy.

2. Verfahren zur Herstellung von Carbamoylcarbonsäureamiden der allgemeinen Formel I gemäß Anspruch 1, dadurch gekennzeichnet, daß man eine Carbamoylcarbonsäure der allgemeinen Formel II 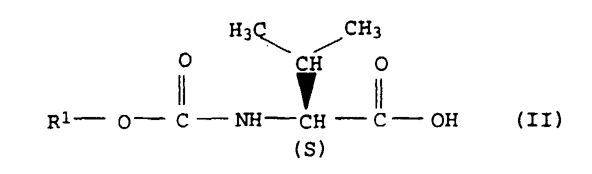 mit einem Amin der allgemeinen Formel III 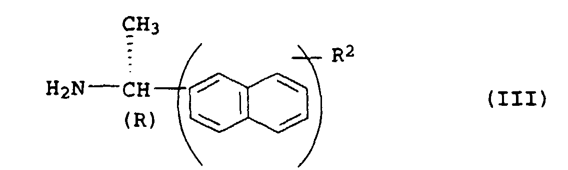 umsetzt.

3. Verfahren zur Herstellung von Carbamoylcarbonsäureamiden der allgemeinen Formel I gemäß Anspruch 1, dadurch gekennzeichnet, daß man
a) ein Carbamoylcarbonsäureamid der allgemeinen Formel I 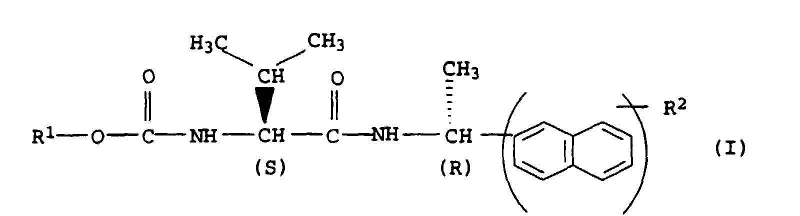 in der die Gruppe R¹-O-(CO) für eine Schutzgruppe steht, die in an sich bekannter Weise abgespalten werden kann, in ein Aminosäureamid IV 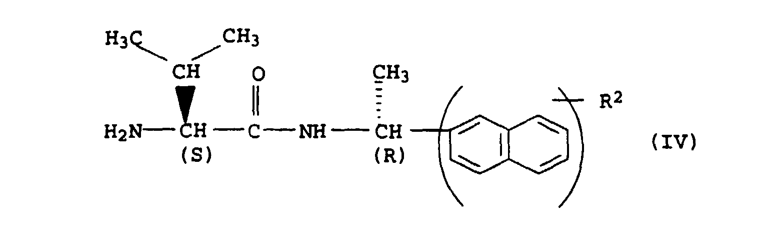 überführt und
b) das so erhaltene Aminosäureamid IV mit einem Chlorameisensäureester der allgemeinen Formel V 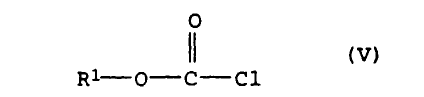 in Gegenwart einer Base umsetzt.

4. Zur Bekämpfung von Schadpilzen geeignete Mittel, enthaltend eine wirksame Menge mindestens einer Verbindung der allgemeinen Formel I gemäß Anspruch 1 und mindestens ein übliches Formulierungshilfsmittel.

5. Verfahren zur Herstellung der Mittel gemäß Anspruch 3, dadurch gekennzeichnet, daß man eine fungizid wirksame Menge mindestens einer Verbindung der allgemeinen Formel I gemäß Anspruch 1 und mindestens ein übliches Formulierungshilfsmittel in an sich bekannter Weise miteinander verarbeitet.

6. Verfahren zur Bekämpfung von Schadpilzen, dadurch gekennzeichnet, daß man die Schadpilze, deren Lebensraum oder die von ihnen freizuhaltenden Pflanzen, Räume, Flächen oder Materialien mit einer wirksamen Menge mindestens einer Verbindung der allgemeinen Formel I gemäß Anspruch 1 oder mit einem Mittel gemäß Anspruch 3 behandelt.

7. Verwendung der Verbindungen der allgemeinen Formel I gemäß Anspruch 1 oder der Mittel gemäß Anspruch 3 zur Bekämpfung von Schadpilzen.

## Claims

1. A carbamoylcarboxamide of the general formula I 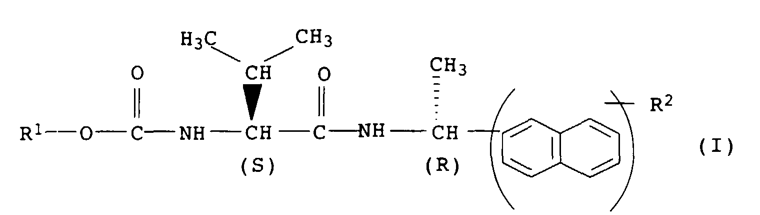 in an isomeric purity of more than 90% by weight where the variables have the following meanings:
R¹ is C₁-C₈-alkyl, C₂-C₈-alkenyl or C₂-C₈-alkynyl,
it being possible for these radicals to be partially or fully halogenated and/or to have attached to them one to three of the following groups: cyano, C₁-C₄-alkoxy, C₁-C₄-haloalkoxy, C₁-C₄-alkylthio, C₁-C₄-alkoxycarbonyl, C₃-C₇-cycloalkyl, C₃-C₇-cycloalkenyl, aryl, aryloxy and hetaryl,
it being possible for the cyclic and aromatic rings of these groups, in turn, to have attached to them one to three of the following substituents: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl, aryloxy and hetaryl;
R² is hydrogen, halogen, cyano, nitro, C₁-C₈-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-haloalkylthio or or a phenyl group bonded via oxygen or sulfur which is unsubstituted or can have attached to it one to three of the following substituents: halogen, C₁-C₄-alkyl and C₁-C₄-alkoxy.

2. A process for the preparation of a carbamoylcarboxamide of the general formula I as claimed in claim 1, which comprises reacting a carbamoylcarboxylic acid of the general formula II 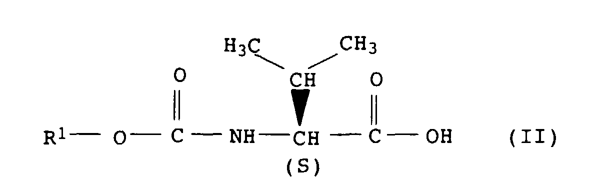 with an amine of the general formula III.

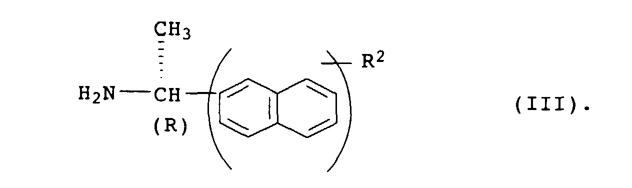

3. A process for the preparation of a carbamoyl carboxamide of the general formula I as claimed in claim 1, which comprises
a) converting a carbamoylcarboxamide of the general formula I 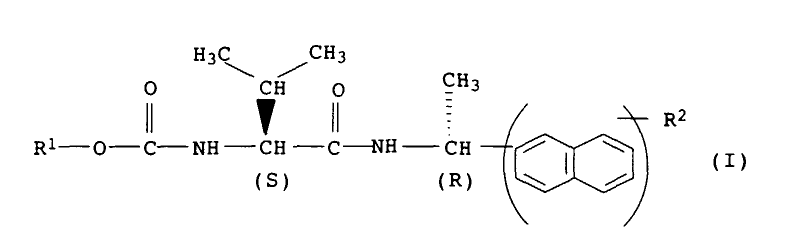 where the group R¹-O-(CO) is a protective group which can be eliminated in a manner known per se into an amino acid amide IV 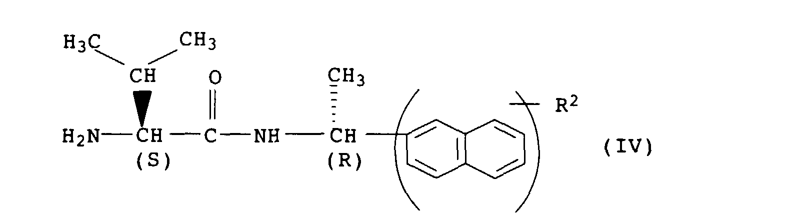 and
b) reacting the resulting amino acid amide IV with a chloroformic ester of the general formula V 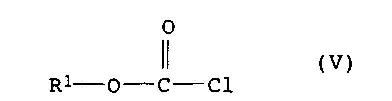 in the presence of a base.

4. A composition suitable for controlling harmful fungi, comprising an effective amount of at least one compound of the general formula I as claimed in claim 1 and at least one customary formulation auxiliary.

5. A process for the preparation of a composition as claimed in claim 4, which comprises processing a fungicidally active amount of at least one compound of the general formula I as claimed in claim 1 together with at least one customary formulation auxiliary in a manner known per se.

6. A method of controlling harmful fungi, which comprises treating the harmful fungi, their environment, or the plants, spaces, areas or materials to be kept free from them, with an effective amount of at least one compound of the general formula I as claimed in claim 1 or with a composition as claimed in claim 4.

7. The use of a compound of the general formula I as claimed in claim 1 or of a composition as claimed in claim 4 for controlling harmful fungi.

## Revendications

1. Carbamoylcarboxamides de formule générale I 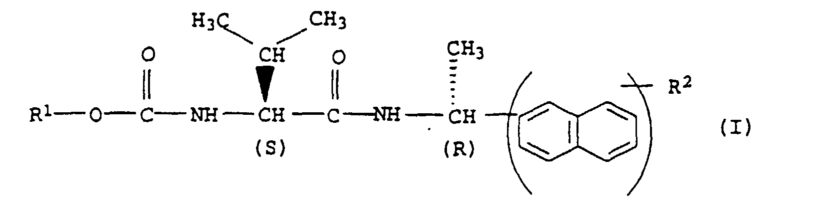 à une pureté en isomère supérieure à 90 % en poids, les symboles de la formule ayant les significations suivantes
R1 : un groupe alkyle en C1-C8, alcényle en C2-C8 ou alcynyle en C2-C8, ces groupes pouvant être partiellement ou totalement halogénés et/ou porter un à trois des substituants suivants : cyano, alcoxy en C1-C4, halogénoalcoxy en C1-C4, alkylthio en C1-C4, (alcoxy en C1-C4)carbonyle, cycloalkyle en C3-C7, cycloalcényle en C3-C7, aryle, aryloxy et hétéroaryle,
les noyaux cycliques et aromatiques de ces groupes pouvant eux-mêmes porter un à trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle, aryloxy et hétéroaryle ;
R2 : l'hydrogène, un halogène, un groupe cyano, nitro, alkyle en C1-C8, (alcoxy en C1-C4)alkyle, halogénoalkyle en C1-C4, alcoxy en C1-C4, halogénoalcoxy en C1-C4, alkylthio en-C1-C4, halogénoalkylthio en C1-C4, ou un groupe phényle relié par l'oxygène ou le soufre, qui est non substitué ou peut porter un à trois des substituants suivants : halogéno, alkyle en C1-C4 et alcoxy en C1-C4.

2. Procédé de préparation des carbamoylcarboxamides de formule générale I de la revendication 1, caractérisé par le fait que l'on fait réagir un acide carbamoylcarboxylique de formule générale II 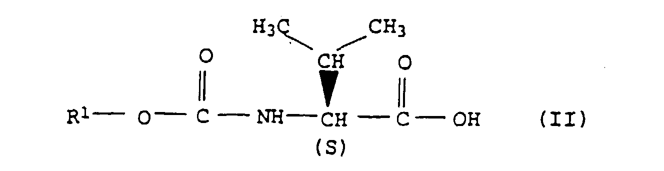 avec une amine de formule générale III

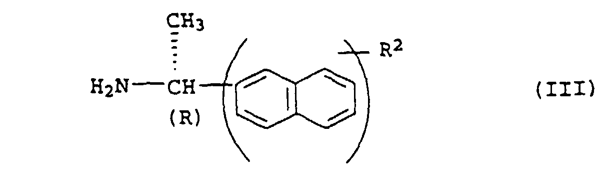

3. Procédé de préparation des carbamoylcarboxamides de formule générale I de la revendication 1, caractérisé par le fait que
a) on convertit un carbamoylcarboxamide de formule générale I 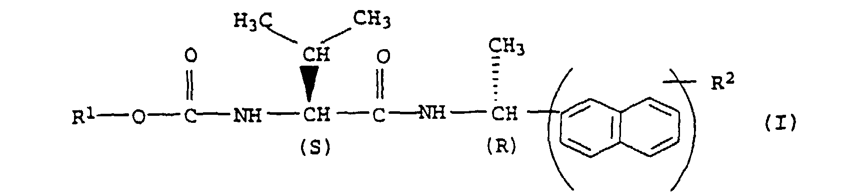 dans laquelle le groupe R1-O-(CO) est un groupe protecteur qu'on peut scinder de manière connue en soi, en un amide d'aminoacide IV 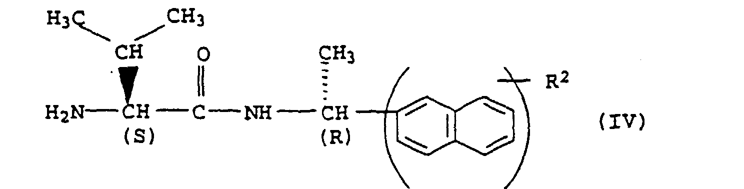 et
b) on fait réagir cet amide d'aminoacide IV avec un ester chloroformique de formule générale V 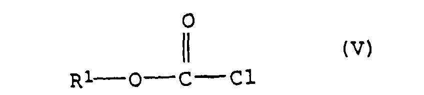 en présence d'une base.

4. Produit utilisable pour la lutte contre les mycètes nuisibles, contenant une quantité efficace d'au moins un composé de formule générale I selon revendication 1 et au moins un produit auxiliaire usuel de formulation.

5. Procédé de préparation des produits de la revendication 3, caractérisé par le fait que l'on combine entre eux, de manière connue en soi, une quantité fongicide efficace d'au moins un composé de formule générale I de la revendication 1 et au moins un produit auxiliaire usuel de formulation.

6. Procédé pour combattre les mycètes nuisibles, caractérisé par le fait que l'on traite les mycètes nuisibles, leur habitat ou les végétaux, locaux, aires ou matériaux qu'on veut protéger contre les mycètes par une quantité efficace d'au moins un composé de formule générale I de la revendication 1 ou par un produit selon revendication 3.

7. Utilisation des composés de formule générale I de la revendication 1 ou des produits de la revendication 3 pour la lutte contre les mycètes nuisibles.
